# EUROPEAN PATENT APPLICATION

(11) **EP 4 563 147 A1**
(43) Date of publication of application: **04.06.2025**
(21) Application number: 23846668.4
(22) Date of filing: 28.07.2023
(51) Int. Cl.: A61K 31/454, A61K 9/20, A61K 9/48, A61K 47/22, A61K 47/32, A61K 47/38, A61P 31/04, A61P 31/06

(54) **DELAMANID-CONTAINING SOLID DISPERSION**

(30) Priority: 29.07.2022 JP 2022122027
(71) Applicant: Otsuka Pharmaceutical Co., Ltd., Tokyo 101-8535 (JP)
(72) Inventor: KAWASAKI, Junichi, Osaka-shi, Osaka 540-0021 (JP); NAKAMURA, Atsuya, Osaka-shi, Osaka 540-0021 (JP)
(74) Representative: Hoffmann Eitle
(86) International application number: PCT/JP2023/027723
(87) International publication number: WO 2024/024938

(57) **Abstract**

Disclosed is a solid dispersion comprising the following components (a), (b), and (c):
(a) delamanid;
(b) at least one member selected from the group consisting of polyvinyl caprolactam-polyvinyl acetate-polyethylene glycol graft copolymers and vinyl pyrrolidone-vinyl acetate copolymers; and
(c) at least one member selected from the group consisting of hydroxypropyl methylcellulose phthalates and hydroxypropyl methylcellulose acetate succinates.

## Description

### Technical Field

The present invention relates to a solid dispersion comprising delamanid and a method for producing the solid dispersion. All of the documents disclosed in the present specification are incorporated herein by reference.

### Background Art

Delamanid is (2R)-2-methyl-6-nitro-2-[(4-{4-[4-(trifluoromethoxy)phenoxy]piperidin-1-yl}phenoxy)methyl]-2,3-dihydroimidazo[2,1-b]oxazole represented by the following formula: 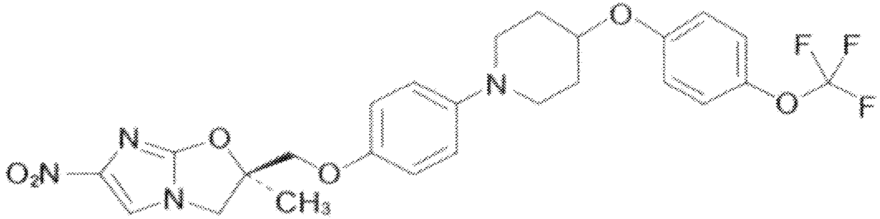 Delamanid has excellent bactericidal action against Mycobacterium tuberculosis, multidrug-resistant Mycobacterium tuberculosis, and atypical mycobacteria (PTL 1). Delamanid is known as a poorly water-soluble compound, which has low solubility in water; thus, efforts have been made to improve solubility and oral absorbability (PTL 2 and PTL 3).

### Citation List

### Patent Literature

PTL 1: JP2004-149527A
PTL 2: WO2007/013477
PTL 3: WO2019/240104

### Summary of Invention

### Technical Problem

In order to improve the solubility of poorly water-soluble compounds, various solubilization techniques have been used, such as micronization (formation into nanoparticles), metastable forms (crystal polymorphs), salts, cocrystals, amorphous forms, and solubilization using, for example, surfactants and oils. Among these, amorphous forms are a highly practical formulation technology; one approach to improve the instability of amorphous forms includes a solid dispersion technology, which involves complexing with a carrier, such as a polymer. A solid dispersion refers to a dispersion of one or more active ingredients in an inert carrier or a matrix thereof in the solid state. Methods for producing a solid dispersion include coprecipitation, spray drying, hot-melt extrusion, and the like. Hot-melt extrusion (HME) is applicable to continuous processes (which can be easily scaled up), solvent-free methods (which are useful from environmental and cost perspectives since, for example, no treatment for solvents would be required and no residual solvents would be present), and different drug delivery systems (including granules, pellets, sustained-release tablets, suppositories, stents, ophthalmic inserts, and transdermal and transmucosal delivery systems), and has many other advantages. However, because the process parameters for HME depend on thermodynamic and rheological properties, a high treatment temperature is typically required, which poses the risk of thermal degradation of drugs. Lowering the treatment temperature to reduce the risk poses concerns in terms of residues of crystalline drugs. Delamanid is a compound with poor thermal stability (melting point: about 195°C (decomposition)), and its melting point and decomposition point are close to each other. Therefore, it is thought that its production by HME would be difficult since HME requires high-temperature treatment, which would result in generation of decomposition products.

An object of the present invention is to provide a solid dispersion comprising delamanid, a method for producing the solid dispersion, a pharmaceutical composition comprising the solid dispersion, and an oral solid formulation comprising the solid dispersion.

### Solution to Problem

In order to solve the above problems, the present inventors conducted extensive research and found that a solid dispersion having excellent solubility etc. can be obtained by combining delamanid, a specific vinyl polymer, and a specific cellulose compound. The present inventors conducted further research based on such findings and completed the present invention.

The present invention encompasses, for example, the subject matter described in the following Items.

### Item 1.

A solid dispersion comprising the following components (a), (b), and (c):
(a) delamanid;
(b) at least one member selected from the group consisting of polyvinyl caprolactam-polyvinyl acetate-polyethylene glycol graft copolymers and vinyl pyrrolidone-vinyl acetate copolymers; and
(c) at least one member selected from the group consisting of hydroxypropyl methylcellulose phthalates and hydroxypropyl methylcellulose acetate succinates.

### Item 2.

The solid dispersion according to Item 1, wherein the mass ratio of component (a) to component (b) is within the range of 1:1 to 1:20.

### Item 3.

The solid dispersion according to Item 1 or 2, wherein the mass ratio of component (a) to component (b) is within the range of 1:1 to 1:8.

### Item 4.

The solid dispersion according to any one of Items 1 to 3, wherein the mass ratio of component (a) to component (b) is within the range of 1:1 to 1:5.

### Item 5.

The solid dispersion according to any one of Items 1 to 4, wherein the mass ratio of component (a) to component (c) is within the range of 1:1 to 1:10.

### Item 6.

The solid dispersion according to any one of Items 1 to 5, wherein the mass ratio of component (a) to component (c) is within the range of 1:1 to 1:8.

### Item 7.

The solid dispersion according to any one of Items 1 to 6, wherein the mass ratio of component (a) to component (c) is within the range of 1:1 to 1:5.

### Item 8.

The solid dispersion according to any one of Items 1 to 7, wherein the mass ratio of component (a) to component (b) is within the range of 1:1 to 1:5, and the mass ratio of component (a) to component (c) is within the range of 1:1 to 1:5.

### Item 9.

The solid dispersion according to any one of Items 1 to 8, wherein component (b) comprises a polyvinyl caprolactam-polyvinyl acetate-polyethylene glycol graft copolymer.

### Item 10.

The solid dispersion according to any one of Items 1 to 9, wherein component (b) comprises a vinyl pyrrolidone-vinyl acetate copolymer.

### Item 11.

The solid dispersion according to any one of Items 1 to 10, wherein component (c) comprises a hydroxypropyl methylcellulose phthalate.

### Item 12.

The solid dispersion according to any one of Items 1 to 11, wherein component (b) comprises a polyvinyl caprolactam-polyvinyl acetate-polyethylene glycol graft copolymer, and component (c) comprises a hydroxypropyl methylcellulose phthalate.

### Item 13.

The solid dispersion according to any one of Items 1 to 12, wherein component (b) comprises a vinyl pyrrolidone-vinyl acetate copolymer, and component (c) comprises a hydroxypropyl methylcellulose phthalate.

### Item 14.

The solid dispersion according to any one of Items 1 to 13, further comprising vitamin E.

### Item 15.

The solid dispersion according to Item 14, wherein the vitamin E is dl-α-tocopherol.

### Item 16.

The solid dispersion according to any one of Items 1 to 15, which is a hot-melt extrudate or a ground material thereof.

### Item 17.

A pharmaceutical composition comprising the solid dispersion of any one of Items 1 to 16.

### Item 18.

An oral solid formulation comprising the solid dispersion of any one of Items 1 to 16.

### Item 19.

The oral solid formulation according to Item 18, which is a tablet or a capsule.

### Item 20.

A method for producing the solid dispersion of any one of Items 1 to 16, comprising subjecting a composition comprising components (a), (b), and (c) to hot-melt extrusion.

### Item 21.

The solid dispersion according to any one of Items 1 to 16, which does not comprise xylitol.

### Item 22.

The solid dispersion according to any one of Items 1 to 16, which does not comprise a sugar alcohol.

### Item 23.

The solid dispersion according to any one of Items 1 to 16, which does not comprise xylitol, sorbitol, mannitol, and a poloxamer.

### Item 24.

The solid dispersion according to Item 9, 11, or 12, wherein the mass ratio of component (a) to component (b) to component (c) is about 1:4:3.

### Item 25.

The solid dispersion according to Item 10, 11, or 13, wherein the mass ratio of component (a) to component (b) to component (c) is about 1:2:2.

### Advantageous Effects of Invention

The solid dispersion comprising delamanid encompassed by the present invention has excellent solubility etc. Furthermore, the method for producing a delamanid solid dispersion encompassed by the present invention enables the production in an inexpensive manner with less environmental impact.

### Brief Description of Drawings

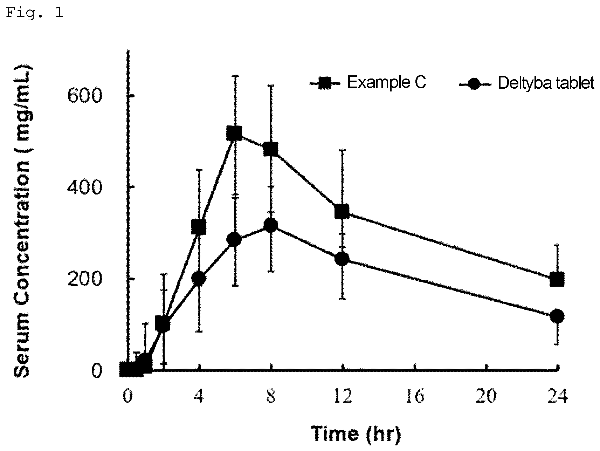

Fig. 1 is a graph showing changes over time of the serum concentration after administration of a capsule comprising the solid dispersion of Example C to dogs.

### Description of Embodiments

In the present specification, the term "about" means to encompass an error, for example, a variation within ±10%, within ±5%, or within ±1% of the subsequent numerical value.

In the present specification, the terms "comprise" and "contain" encompass the concepts of consisting essentially of and consisting of.

Embodiments of the present invention are described in further detail below.

### Solid Dispersion

The solid dispersion encompassed by the present invention (which may be referred to below as "the solid dispersion of the present invention") comprises (a) delamanid, (b) at least one member selected from the group consisting of polyvinyl caprolactam-polyvinyl acetate-polyethylene glycol graft copolymers and vinyl pyrrolidone-vinyl acetate copolymers, and (c) at least one member selected from the group consisting of hydroxypropyl methylcellulose phthalates and hydroxypropyl methylcellulose acetate succinates.

### Component (a)

In the solid dispersion of the present invention, delamanid is preferably in a microcrystalline or amorphous form. Specifically, it is preferable that no crystalline peaks are observed in the X-ray powder diffraction patterns. The X-ray powder diffraction can be measured according to "General Tests, Processes and Apparatus, 2.58 X-ray Powder Diffraction Method" of the Japanese Pharmacopoeia (18th edition), and can also be measured, for example, under the following conditions:
Measuring device: X'Pert PRO MPD (Spectris Co., Ltd.)
Optical system: Concentrated optical system (transmission method)
Goniometer radius: 240 mm
Tube voltage, tube current: 45 kV, 40 mA
Incident slit: 0.04 rad solar slit
Divergence slit: 1/2 deg
Light-receiving slit: 0.04 rad large solar slit
Anti-scatter slit: 5.5 mm
Scanning range: 2θ 3 to 40 deg
Operation speed: 1.11 deg/sec
Sampling interval: 0.02 deg/step
Wobbled scan: step count 5, step size 0.02 deg

Delamanid can be produced by conventional methods. Examples of the methods include the methods described in JP2004-149527A and US Patent Application Publication No. 2006/094767.

### Component (b)

The present inventors found that a combined use of component (a) and component (b) improves compatibility between these two components, makes hot-melt kneading possible, ensures fluidity in a melt kneader, achieves excellent production of a solid dispersion, and produces a solid dispersion with solubility equal to or better than that of a delamanid drug substance (jetmilled product).

Of components (b), the polyvinyl caprolactam-polyvinyl acetate-polyethylene glycol graft copolymer (which may be referred to below as "component (b1)") comprises at least one polyvinyl caprolactam block, at least one polyvinyl acetate block, and at least one polyethylene glycol block. The polyethylene glycol may be, for example, PEG6000. The ratio of polyvinyl caprolactam block/polyvinyl acetate block/polyethylene glycol block may be, for example, about 57/30/13.

In one embodiment, component (b1) may be a copolymer represented by the following formula: 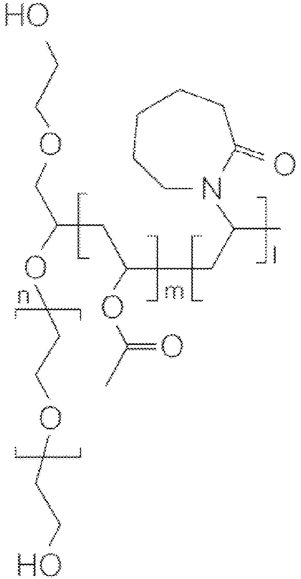 In the formula, 1, m, and n are each independently an integer of 1 or more.

The average molecular weight of component (b1) can be, for example, 10000 g/mol or more, 50000 g/mol or more, or 90000 g/mol or more. The average molecular weight of component (b1) can be, for example, 1000000 g/mol or less, 500000 g/mol or less, 200000 g/mol or less, or 140000 g/mol or less. The average molecular weight of component (b1) can be within the range of any combination of the above lower limits and upper limits, and can be, for example, within the range of 10000 to 1000000 g/mol, and preferably within the range of 90000 to 140000 g/mol.

In one embodiment, component (b1) preferably comprises Soluplus (trademark) from BASF.

Of components (b), the vinyl pyrrolidone-vinyl acetate copolymer (which may be referred to below as "component (b2)") is not particularly limited as long as it is a copolymer having at least vinyl pyrrolidone (usually 1-vinyl-2-pyrrolidone) and vinyl acetate as polymerization components. The copolymerization ratio (mass ratio) of vinyl pyrrolidone to vinyl acetate can be, for example, within the range of 1:9 to 9:1, within the range of 2:8 to 8:2, or within the range of 3:7 to 7:3. The copolymerization ratio (mass ratio) of vinyl pyrrolidone to vinyl acetate can be, for example, within the range of 4:6 to 8:2, or within the range of 5:5 to 7:3.

The sequence of monomers of component (b2) is not particularly limited, and component (b2) may be any of a random copolymer, an alternating copolymer, a block copolymer, and a graft copolymer. In one embodiment, component (b2) is preferably a random copolymer.

The average molecular weight of component (b2) can be, for example, 10000 g/mol or more, 20000 g/mol or more, 30000 g/mol or more, 40000 g/mol or more, or 45000 g/mol or more. The average molecular weight of component (b2) can be, for example, 1000000 g/mol or less, 500000 g/mol or less, 100000 g/mol or less, or 70000 g/mol or less. The average molecular weight of component (b2) can be within the range of any combination of the above lower limits and upper limits, and can be, for example, within the range of 10000 to 1000000 g/mol, and preferably within the range of 45000 to 70000 g/mol.

In one embodiment, component (b2) preferably comprises copolyvidone, such as Kollidon (trademark) VA 64 from BASF.

In one embodiment, component (b) preferably comprises component (b1). In another embodiment, component (b) preferably comprises component (b2). In yet another embodiment, component (b) is preferably a combination of component (b1) and component (b2). In this combination, the mass ratio of component (b1) to component (b2) is not particularly limited and is, for example, within the range of 0.5:1 to 10:1, and preferably within the range of 1:1 to 6:1.

### Component (c)

The present inventors found that a solid dispersion with even better solubility can be obtained by further combining component (c) with components (a) and (b).

Of components (c), the hydroxypropyl methylcellulose phthalate (which may be referred to below as "component (c1)") is a polymer compound in which phtalic acid is half-esterified with hydroxypropyl cellulose. The hydroxypropyl methylcellulose phthalate is preferably "hypromellose phthalate" listed in the Official Monographs (e.g., chemical drugs) in the Japanese Pharmacopoeia (18th edition). Hydroxypropyl methylcellulose phthalates are commercially available, for example, from Shin-Etsu Chemical Co., Ltd. under the trade names of HPMCP (trademark) HP-55, HP-55S, and HP-50. In the present invention, any of these commercially available products can be used. In one embodiment, the hydroxyl groups of the hydroxypropyl methylcellulose phthalate are preferably substituted with at least 21 to 35 mass% (e.g., 21 to 27 mass% or 27 to 35 mass%) of carboxybenzoyl groups. In this embodiment, it is preferable that the hydroxyl groups of the hydroxypropyl methylcellulose phthalate are substituted with 18 to 24 mass% of methoxy groups, 5 to 10 mass% of hydroxypropoxy groups, and 21 to 35 mass% of carboxybenzoyl groups.

Of components (c), the hydroxypropyl methylcellulose acetate succinate (which may be referred to below as component (c2)) is a polymer compound in which acetic acid and succinic acid are esterified with hydroxypropyl cellulose. The hydroxypropyl methylcellulose acetate succinate is preferably "hypromellose acetate succinate" listed in the Official Monographs (e.g., chemical drugs) in the Japanese Pharmacopoeia (18th edition). Hydroxypropyl methylcellulose acetate succinates are commercially available, for example, from Shin-Etsu Chemical Co., Ltd. under the trade names of Shin-Etsu AQOAT (trademark) AS-LF, AS-MF, and AS-HF. In the present invention, any of these commercially available products can be used. The hydroxyl groups of the hydroxypropyl methylcellulose acetate succinate are preferably substituted with 20 to 26 mass% of methoxy groups, 5 to 10 mass% of hydroxypropoxy groups, 5 to 14 mass% of acetyl groups, and 4 to 18 mass% of succinoyl groups.

In one embodiment, component (c) preferably comprises component (c1) (for example, component (c1) alone, or a combination of component (c1) and component (c2)).

In the solid dispersion of the present invention, the content of component (a) can be, for example, 1 mass% or more, 5 mass% or more, or 10 mass% or more. The content of component (a) can be, for example, 30 mass% or less, 25 mass% or less, or 20 mass% or less. The content of component (a) can be, for example, within the range of any combination of the above lower limits and upper limits, and can be, for example, within the range of 1 to 30 mass%.

The mass ratio of component (a) to component (b) is not particularly limited. The mass ratio of component (a) to component (b) may be 1:less than 1 (for example, within the range of 1:0.1 to 1:0.9), but is usually 1:1 or more (i.e., component (b)/component (a) being 1/1 or more), preferably 1:1.5 or more, and more preferably 1:2 or more. The mass ratio of component (a) to component (b) can be, for example, 1:20 or less (i.e., component (b)/component (a) being 20/1 or less), 1:15 or less, 1:10 or less, 1:8 or less, or 1:5 or less. The mass ratio of component (a) to component (b) is within the range of any combination of the above lower limits and upper limits, and is, for example, within the range of 1:1 to 1:20, preferably within the range of 1:1 to 1:8, more preferably within the range of 1:1 to 1:5, even more preferably within the range of 1:2 to 1:5, and still more preferably within the range of 1:2 to 1:4, or about 1:2 or about 1:4. When component (b) comprises component (b1), the mass ratio of component (a) to component (b) is preferably about 1:4, and when component (b) comprises component (b2), the mass ratio of component (a) to component (b) is preferably about 1:2.

The mass ratio of component (a) to component (c) can be 1:less than 1 (for example, within the range of 1:0.1 to 1:0.9), but is usually 1:1 or more (i.e., component (c)/component (a) being 1/1 or more), preferably 1:1.5 or more, and more preferably 1:2 or more. The mass ratio of component (a) to component (c) can be, for example, 1:10 or less (i.e., component (c)/component (a) being 10/1 or less), 1:8 or less, or 1:5 or less. The mass ratio of component (a) to component (c) is within the range of any combination of the above lower limits and upper limits, and is, for example, within the range of 1:1 to 1:10, preferably within the range of 1:1 to 1:8, more preferably within the range of 1:1 to 1:5, even more preferably within the range of 1:1 to 1:4, still more preferably within the range of 1:2 to 1:4, and particularly preferably about 1:2 or about 1:3. When component (b) comprises component (b1), the mass ratio of component (a) to component (c) is preferably about 1:3, and when component (b) comprises component (b2), the mass ratio of component (a) to component (c) is preferably about 1:2.

In one embodiment, the mass ratio of component (a) to component (b) to component (c) is preferably within the range of 1:1 to 8:1 to 8, more preferably within the range of 1:1 to 5:1 to 5, even more preferably within the range of 1:1.5 to 5:1.5 to 5, still more preferably within the range of 1:2 to 4:2 to 4, or about 1:2:2 or about 1:4:3. When component (b) comprises component (b1), the mass ratio of component (a) to component (b) to component (c) is preferably about 1:4:3, and when component (b) comprises component (b2), the mass ratio of component (a) to component (b) to component (c) is preferably about 1:2:2.

### Other Components

The solid dispersion of the present invention can further comprise other components. Examples of the other components include antioxidants, pH-independent water-soluble polymer compounds other than component (b), and plasticizers. These other components may be used alone or in a combination of two or more.

Examples of antioxidants include vitamin E, dibutylhydroxytoluene, butylhydroxyanisole, soybean lecithin, ascorbyl palmitate, cysteine hydrochloride, ascorbic acid, citric acid, erythorbic acid, sodium nitrite, sodium sulfite, sodium thioglycolate, and sodium thiomalate. In one embodiment, it is preferable that the antioxidant comprises vitamin E, and it is also preferable that the antioxidant comprises vitamin E and dibutylhydroxytoluene (for example, the mass ratio of vitamin E to dibutylhydroxytoluene is 15:1 to 1:1). According to this embodiment, the formation of decomposition products during storage (in particular, during storage at a temperature as high as about 60°C) can be suppressed.

Examples of vitamin E include natural and synthetic vitamin E, such as d-α-tocopherol, d-δ-tocopherol, d-α-tocopherol acetate, d-α-tocopherol succinate, dl-α-tocopherol, dl-α-tocopherol acetate, dl-α-tocopherol succinate, dl-α-tocopherol calcium succinate, and dl-α-tocopherol nicotinate. Vitamin E is preferably dl-α-tocopherol, dl-α-tocopherol acetate, dl-α-tocopherol succinate, or dl-α-tocopherol nicotinate, and more preferably dl-α-tocopherol.

The amount of the antioxidant (e.g., vitamin E) blended in the solid dispersion of the present invention is usually 0.001 parts by mass or more, preferably 0.01 parts by mass or more, and more preferably 0.03 parts by mass or more, per part by mass of delamanid. The amount of the antioxidant (e.g., vitamin E) is usually 1 part by mass or less, preferably 0.8 parts by mass or less, and more preferably 0.5 parts by mass or less, per part by mass of delamanid. The amount of the antioxidant (e.g., vitamin E) is within the range of any combination of the above lower limits and upper limits, and is, for example, usually within the range of 0.001 to 1 part by mass, preferably within the range of 0.01 to 0.8 parts by weight, and more preferably within the range of 0.03 to 0.5 parts by mass, per part by mass of delamanid.

Examples of pH-independent water-soluble polymer compounds other than component (b) include hydroxypropyl cellulose, hydroxypropyl methylcellulose, polyvinylpyrrolidone, polyethylene glycol, and cyclodextrin.

Examples of plasticizers include triethyl citrate, glycerin, glycerin fatty acid esters, medium-chain fatty acid triglycerides, triacetin, castor oil, propylene glycol, and polysorbates.

The other components, such as the pH-independent water-soluble polymer compound other than component (b) and the plasticizer, can also be blended in the solid dispersion in the same amounts as that of the antioxidant.

In one embodiment, the solid dispersion of the present invention preferably does not comprise xylitol, more preferably does not comprise xylitol, sorbitol, and mannitol, and further preferably does not comprise a sugar alcohol.

In one embodiment, the solid dispersion of the present invention preferably does not comprise a poloxamer (a block copolymer comprising a polyoxypropylene chain and two polyoxyethylene chains sandwiching the polyoxypropylene chain).

In one embodiment, the solid dispersion of the present invention preferably does not comprise xylitol, sorbitol, mannitol, and a poloxamer.

The solid dispersion of the present invention is preferably a hot-melt extrudate or a ground material thereof, and is preferably produced by the production method described below. The ground material is usually in the form of particles and may be referred to as solid dispersion particles, molten granules, or the like. The size of the ground material is not particularly limited. For example, the size is such that it passes through a sieve with an aperture of 500 µm (30 mesh) or 425 µm (36 mesh), and preferably a sieve with an aperture of 355 µm (42 mesh), 300 µm (50 mesh), 250 µm (60 mesh), 212 µm (70 mesh), 180 µm (83 mesh), 160 µm (93 mesh), 150 µm (100 mesh), 125 µm (119 mesh), 106 µm (140 mesh), 100 µm (149 mesh), 90 µm (166 mesh), 75 µm (200 mesh), 63 µm (235 mesh), 53 µm (280 mesh), 45 µm (330 mesh), 38 µm (390 mesh), 32 µm (440 mesh), or 25 µm (500 mesh). The particle size may also be adjusted by any two different sieves from the above sieves, and the size of the ground material is, for example, 25 to 500 µm, preferably 53 to 150 µm, and more preferably 75 to 106 µm.

### Method for Producing Solid Dispersion

The method for producing the solid dispersion encompassed by the present invention (which may be referred to below as "the method for producing the solid dispersion of the present invention") preferably comprises subjecting a composition comprising components (a), (b), and (c) to hot-melt extrusion (HME). The composition may comprise components other than components (a), (b), and (c), and may also comprise those listed in the "Other Components" in the "Solid Dispersion" section above. HME is a method typically characterized by hot-melting starting materials while uniformly mixing, followed by extrusion and then cooling, and can be carried out by using a conventional method and apparatus (e.g., a stirrer or kneader having a heat source) .

For HME, for example, an extruder with a screw in the barrel (cylinder) (for example, a single-screw extruder, a twin-screw extruder) can be used. Among these, a twin-screw extruder is preferred.

An extruder includes five main parts: a hopper (the inlet structure), a motor (which controls the screw rotation), a screw (the primary power source for shearing and moving the material), a barrel (which houses the screw and provides temperature control), and a die (exit) (which controls the shape and size of the extruded molded product).

Starting materials are fed from the extruder hopper into the apparatus maintained at an appropriate heating and melting temperature. By rotating the screw, the solid materials in the starting materials are melted and uniformly kneaded. Alternatively, the starting materials may be preliminarily mixed before being fed into the apparatus, if necessary.

In the production method of the present invention, the conditions, such as the pressure, temperature, powder supply rate, die orifice diameter, screw shape, and screw rotation speed, vary depending on the type etc. of the extruder. It is preferable to combine each of these conditions so that the temperature is equal to or lower than the decomposition temperature of the starting materials. In particular, the heating temperature must be appropriately set from the standpoint of stability issues, such as decomposition or denaturation of the starting materials. The heating temperature is preferably 190°C or lower, more preferably 185°C or lower, and even more preferably 180°C or lower. The heating temperature is usually 150°C or higher, preferably 155°C or higher, and more preferably 160°C or higher. The heating temperature can be within the range of any combination of the above lower limits and upper limits, and can be, for example, within the range of 150 to 190°C, and preferably within the range of 160 to 190°C. The screw rotation speed is preferably 10 rpm or more, more preferably 15 rpm or more, and even more preferably 20 rpm or more. The screw rotation speed is preferably 250 rpm or less, more preferably 200 rpm or less, even more preferably 150 rpm or less, and still more preferably 100 rpm or less. The screw rotation speed can be within the range of any combination of the above lower limits and upper limits, and can be, for example, within the range of 20 to 250 rpm.

A preferred method for producing the solid dispersion of the present invention comprises a step of mixing component (a), component (b), and component (c), and optionally additives to prepare a composition (mixture), and a step of subjecting the composition to hot-melt extrusion. In the hot-melt extrusion step, the composition is preferably fed into a twin-screw extruder and treated at a screw rotation speed of 20 to 250 rpm at a treatment temperature of 160 to 190°C.

The method for producing the solid dispersion of the present invention preferably further comprises a step of grinding the hot-melt extrudate. The use of an appropriate grinder for grinding makes it possible to easily obtain solid dispersion particles having any particle size; these particles can be used directly as powders, fine granules, granules, and the like. By adding suitable components (additives) for pharmaceutical formulations and through formulation processes, the solid dispersion (or solid dispersion particles) of the present invention can also be formed into a pharmaceutical composition or an oral formulation, such as an oral solid formulation, comprising the solid dispersion of the present invention.

### Pharmaceutical Composition

The pharmaceutical composition encompassed by the present invention (which may be referred to below as "the pharmaceutical composition of the present invention") comprises the solid dispersion described above. The amount of the solid dispersion in the pharmaceutical composition of the present invention is not particularly limited and may be, for example, within the range of 50 to 90 mass%. The pharmaceutical composition of the present invention may further comprise other active ingredients and/or additives in addition to the solid dispersion described above. The additives are not particularly limited as long as they are pharmaceutically acceptable. Examples of the additives include excipients, disintegrants, binders, fluidizers, lubricants, preservatives, stabilizers, isotonicity agents, solubilizing agents, coating agents, coloring agents, suspending agents, surfactants, sweeteners, flavoring agents, preservatives, dispersants, and pH adjusters. The additives can be used alone or in a combination of two or more. The amount of other active ingredients and/or additives in the pharmaceutical composition of the present invention is not particularly limited and may be, for example, within the range of 10 to 50 mass%.

Examples of excipients include lactose, anhydrous lactose, refined white sugar, white sugar, D-mannitol, D-sorbitol, xylitol, erythritol, dextrin, crystalline cellulose, microcrystalline cellulose, corn starch, potato starch, and anhydrous calcium hydrogen phosphate.

Examples of disintegrants include sodium carboxymethyl starch, carmellose, carmellose calcium, carmellose sodium, croscarmellose sodium, sodium starch glycolate, crospovidone, low-substituted hydroxypropyl cellulose, and partially pregelatinized starch.

Examples of binders include hydroxypropyl cellulose, hydroxypropyl methyl cellulose, polyvinylpyrrolidone, pregelatinized starch, syrup, and starch syrup.

Examples of fluidizers include light anhydrous silicic acid, calcium silicate, synthetic aluminum silicate, hydrated silicon dioxide, calcium stearate, magnesium aluminometasilicate, and talc.

Examples of lubricants include magnesium stearate, calcium stearate, magnesium silicate, magnesium oxide, talc, hardened oil, sucrose fatty acid ester, and sodium stearyl fumarate.

Examples of coating agents include hydroxypropyl methylcellulose, polyvinyl alcohol, polysorbate, macrogol, and talc.

Examples of coloring agents include yellow ferric oxide, brown iron oxide, red ferric oxide, titanium oxide, Food Blue No. 1, Food Red No. 2, Food Red No. 3, and Food Yellow No. 4.

Examples of suspending agents include polysorbate, polyethylene glycol, gum arabic, glycerin, sucrose fatty acid ester, and gelatin.

Examples of sweeteners include aspartame, sucralose, saccharin, sodium saccharin, starch syrup, and fructose.

Examples of surfactants include sodium lauryl sulfate, polysorbate, and polyoxyethylene hydrogenated castor oil.

The form of the pharmaceutical composition of the present invention is not particularly limited, and the pharmaceutical composition of the present invention is preferably used in the form of, for example, solids (e.g., granules, tablets, or capsules), pellets, or liquids (e.g., suspension). As an example of the pharmaceutical composition of the present invention, the solid dispersion described above and additives as necessary can be filled into a capsule for use as capsules. As another example of the pharmaceutical composition of the present invention, the solid dispersion described above can be mixed with additives as necessary and compressed into tablets for use as tablets. As yet another example of the pharmaceutical composition of the present invention, the solid dispersion described above can be dispersed in water for use as a suspension.

### Oral Formulation

The oral formulation encompassed by the present invention (which may be referred to below as "the oral formulation of the present invention) comprises the solid dispersion described above. The amount of the solid dispersion in the oral formulation of the present invention is not particularly limited, and may be, for example, within the range of 50 to 90 mass%. The oral formulation of the present invention may further comprise other active ingredients and/or additives in addition to the solid dispersion described above. For the additives, those listed as additives in the "Pharmaceutical Composition" section above can be used. The amount of the other active ingredients and/or additives in the oral formulation of the present invention is not particularly limited, and may be, for example, within the range of 10 to 50 mass%. The oral formulation of the present invention may be an oral solid formulation in the form of, for example, tablets, capsules, powders, and granules. In one embodiment, the oral formulation of the present invention is preferably a tablet or a capsule. Tablets include, for example, plain tablets, film-coated tablets, sugar-coated tablets, caplets, and chewable tablets.

Various configurations (properties, structures, functions, etc.) described in the above embodiments of the present invention may be combined in any manner to specify the subject matter encompassed by the present invention. That is, this invention encompasses all of the subject matter comprising any combination of the combinable configurations described herein.

### Examples

The present invention will be described in more detail below; however, the present invention is not limited to the following Examples.

### Examples A1 to A11

Delamanid (hammer mill-ground product, Otsuka Pharmaceutical Co., Ltd.), a polyvinyl caprolactam-polyvinyl acetate-polyethylene glycol graft copolymer (Soluplus (trademark), BASF), hypromellose phthalate (HPMCP (trademark) HP-50, Shin-Etsu Chemical Co., Ltd.), and optionally dl-α-tocopherol (a type of vitamin E; this may be referred to below as "VE") (FUJIFILM Wako Pure Chemical Corporation), and optionally dibutylhydroxytoluene (BHT), were placed in a polyethylene bag in the amounts shown in Tables 1 to 4 and mixed for several minutes.

The resulting mixed powder was molded at an extrusion speed of 40 to 80 rpm using a twin-screw extruder (Pharma 11 twin-screw extruder, Thermo Fisher Scientific) equipped with a die with an orifice diameter of 1 mm by setting the barrel temperature in the kneading section to 160 to 180°C to obtain a rod-shaped molded body. The molded body was cooled at room temperature, finely ground using a grinder (Tablet Smasher HTF-35, Daido Kako Co., Ltd.), and sieved through a 53 µm sieve to obtain particles (solid dispersion particles).

### Example A12

Particles were produced in the same manner as in Example A11, except that the hydroxypropyl methylcellulose phthalate was changed to a hydroxypropyl methylcellulose acetate succinate (Shin-Etsu AQOAT (trademark) AS-LF, Shin-Etsu Chemical Co., Ltd.).

### Comparative Example A1

Particles were produced in the same manner as in Example A1, except that the hydroxypropyl methylcellulose phthalate and dl-α-tocopherol (VE) were not added.

### Comparative Example A2

Particles were produced in the same manner as in Example A3, except that the hydroxypropyl methylcellulose phthalate and dl-α-tocopherol (VE) were not added.

### Comparative Example A3

Particles were produced in the same manner as in Example A11, except that the hydroxypropyl methylcellulose phthalate, dl-α-tocopherol (VE), and dibutylhydroxytoluene (BHT) were not added.

### Comparative Example A4

The same procedures as in Example A1 were performed, except that the polyvinyl caprolactam-polyvinyl acetate-polyethylene glycol graft copolymer and dl-α-tocopherol were not added. As a result, delamanid underwent decomposition during the molding process, and a molded body could not be produced.

### Examples B1 to B5

Delamanid (hammer mill-ground product, Otsuka Pharmaceutical Co., Ltd.), a vinyl pyrrolidone-vinyl acetate copolymer (Kollidon (trademark) VA 64, BASF), a hydroxypropyl methylcellulose phthalate (HPMCP (trademark) HP-50, Shin-Etsu Chemical Co., Ltd.), and dl-α-tocopherol (VE) (FUJIFILM Wako Pure Chemical Corporation) were placed in a polyethylene bag in the amounts shown in Table 5 and mixed for several minutes.

The resulting mixed powder was molded at an extrusion speed of 40 rpm using a twin-screw extruder (Pharma 11 twin-screw extruder, Thermo Fisher Scientific) equipped with a die with an orifice diameter of 1 mm by setting the barrel temperature in the kneading section to 160 to 170°C to obtain a rod-shaped molded body. The molded body was cooled at room temperature, finely ground using a grinder (Tablet Smasher HTF-35, Daido Kako Co., Ltd.), and sieved through a 53 µm sieve to obtain particles (solid dispersion particles).

### Example C

40 g of delamanid (hammer mill-ground product, Otsuka Pharmaceutical Co., Ltd.), 160 g of a polyvinyl caprolactam-polyvinyl acetate-polyethylene glycol graft copolymer (Soluplus (trademark), BASF), 120 g of a hydroxypropyl methylcellulose phthalate (HPMCP (trademark) HP-50, Shin-Etsu Chemical Co., Ltd.), and 9.6 g of dl-α-tocopherol (VE) (FUJIFILM Wako Pure Chemical Corporation) were placed in a polyethylene bag and mixed for several minutes.

The resulting mixed powder was molded at an extrusion speed of 40 rpm using a twin-screw extruder (Pharma 11 twin-screw extruder, Thermo Fisher Scientific) equipped with a die with an orifice diameter of 1 mm by setting the barrel temperature in the kneading section to 180°C to obtain a rod-shaped molded body. The molded body was cooled at room temperature, finely ground using a grinder (Tablet Smasher HTF-35, Daido Kako Co., Ltd.), and sieved through a 53 µm sieve to obtain particles (solid dispersion particles).

### Test Example 1: Solubility

The solubility of the particles of Examples A1 to A12 and Comparative Examples A1 to A3 was examined according to the following Method 1 or 2.

### Method 1

100 mL of a 0.3 mass% aqueous sodium lauryl sulfate solution was placed in a 100 mL beaker and stirred with a magnetic stirrer (rotation speed: 500 rpm). Separately, the particles of the Examples and Comparative Examples were weighed out into an agate mortar so that the amount of delamanid was 5 mg, and several milliliters of the test liquid in the beaker was added. After the mixture was dispersed for about 1 minute, the dispersion was poured into the beaker. Then, 6 mL of the liquid in the beaker was sampled 5 minutes, 10 minutes, 20 minutes, and 30 minutes after the addition of the dispersion, and the liquids were filtered through a membrane filter having a pore size of 0.5 µm or less. The first 2 mL of the filtrate was removed, and the remaining 4 mL of the filtrate was used as a sample solution. The absorbance of the sample solutions was measured at a wavelength of 335 nm.

### Method 2

Sample solutions were produced in the same manner as in Method 1, except that a 0.1 mass% aqueous sodium lauryl sulfate solution was used as the test solution, and the absorbance at a wavelength of 335 nm was measured.

Tables 1 to 4 show the results in terms of the solubility of the particles of Examples A1 to A12 and Comparative Examples A1 to A3.

**Table 1**

| | Delamanid | Soluplus (trademark) | HPMCP (trademark) HP-50 | VE | Barrel temperature (°C) | Extrusion Speed (rpm) | Absorbance (abs) Method 1 | | | |
|---|---|---|---|---|---|---|---|---|---|---|
| | | | | | | | 0 min | 5 min | 10 min | 20 min |
| Comparative Example A1 | 40 g | 120 g | - | - | 170 | 40 | 0 | 0.229 | 0.247 | 0.237 |
| Example A1 | 40 g | 120 g | 40 g | 3.2 g | 170 | 40 | 0 | 0.420 | 0.433 | 0.434 |
| Example A2 | 40 g | 120 g | 80 g | 6.4 g | 170 | 40 | 0 | 0.783 | 0.792 | 0.796 |
| Comparative Example A2 | 40 g | 160 g | - | - | 170 | 40 | 0 | 0.285 | 0.277 | 0.270 |
| Example A3 | 40 g | 160 g | 60 g | 4.8 g | 170 | 40 | 0 | 0.568 | 0.595 | 0.613 |
| Example A4 | 40 g | 160 g | 120 g | 9.6 g | 160→ 170 | 40 | 0 | 0.797 | 0.803 | 0.810 |

**Table 2**

| | Delamanid | Soluplus (trademark) | HPMCP (trademark) HP-50 | VE | BHT | Barrel Temperature (°C) | Extrusion Speed (rpm) | Absorbance (abs) | | | |
|---|---|---|---|---|---|---|---|---|---|---|---|
| | | | | | | | | Method 1 | | | |
| | | | | | | | | 0 min | 5 min | 10 min | 20 min |
| Example A5 | 40 g | 160 g | 40 g | 3.2 g | 0.8 g | 170 | 40 | 0 | 0.416 | 0.441 | 0.451 |
| Example A6 | 40 g | 160 g | 80 g | 6.4 g | 0.8 g | 170 | 40 | 0 | 0.767 | 0.765 | 0.742 |
| Example A7 | 40 g | 160 g | 120 g | 9.6 g | 0.8 g | 170 | 40 | 0 | 0.805 | 0.810 | 0.816 |

**Table 3**

| | Delamanid | Soluplus (trademark) | HPMCP (trademark) HP-50 | VE | Barrel temperature (°C) | Extrusion Speed (rpm) | Absorbance (abs) | | | |
|---|---|---|---|---|---|---|---|---|---|---|
| | | | | | | | Method 1 | | | |
| | | | | | | | 0 min | 10 min | 20 min | 30 min |
| Example A8 | 40 g | 160 g | 120 g | - | 160→ 170 | 80 | 0 | 0.709 | 0.739 | 0.726 |
| Example A9 | 40 g | 160 g | 120 g | 3.2 g | 160→ 170 | 80 | 0 | 0.741 | 0.781 | 0.772 |
| Example A10 | 40 g | 160 g | 120 g | 19.2 g | 160→ 170 | 80 | 0 | 0.782 | 0.781 | 0.784 |

**Table 4**

| | Delamanid | Soluplus (trademark) | HPMCP (trademark) HP-50 | Shin-Etsu AQOAT (trademark) AS-LF | VE | BHT | Barrel Temperature (°C) | Extrusion Speed (rpm) | Absorbance (abs) | | | |
|---|---|---|---|---|---|---|---|---|---|---|---|---|
| | | | | | | | | | Method 2 | | | |
| | | | | | | | | | 0 min | 5 min | 10 min | 20 min |
| Comparative Example A3 | 40 g | 160 g | - | - | - | - | 180 | 40 | 0 | 0.044 | 0.060 | 0.048 |
| Example A11 | 40 g | 160 g | 120 g | - | 9.6 g | 0.8g | 180 | 40 | 0 | 0.647 | 0.673 | 0.678 |
| Example A12 | 40 g | 160 g | - | 120 g | 9.6 g | 0.8 g | 180 | 40 | 0 | 0.336 | 0.355 | 0.339 |

Table 5 shows the results in terms of the solubility of the particles of Examples B1 to B5.

**Table 5**

| | Delamanid | Kollidon (trademark) VA 64 | HPMCP (trademark) HP-50 | VE | Absorbance (abs) | | | |
|---|---|---|---|---|---|---|---|---|
| | | | | | Method 1 | | | |
| | | | | | 0 min | 10 min | 20 min | 30 min |
| Example B1 | 40 g | 40 g | 40 g | 3.2 g | 0 | 0.416 | 0.463 | 0.487 |
| Example B2 | 40 g | 40 g | 80 g | 6.4 g | 0 | 0.550 | 0.566 | 0.577 |
| Example B3 | 40 g | 80 g | 80 g | 6.4 g | 0 | 0.648 | 0.738 | 0.772 |
| Example B4 | 40 g | 120 g | 80 g | 6.4 g | 0 | 0.589 | 0.720 | 0.777 |
| Example B5 | 40 g | 160 g | 80 g | 6.4 g | 0 | 0.566 | 0.708 | 0.748 |

### Test Example 2: Purity of Delamanid

The particles obtained in Examples A8 to A10 were placed in a glass container. After the outer layer was sealed in an aluminum bag, the particles were stored in an environment at a temperature of 60°C for 2 weeks. Using the resulting particles as a sample, the purity of delamanid was examined. The purity of delamanid was determined as follows. Specifically, a column (ACQUITY UPLC (trademark) BEH C18, Nihon Waters K.K.) was connected to UPLC (ultra-high performance liquid chromatography, ACQUITY UPLC/TUV system, Nihon Waters K.K.), the sample cooler was set to 15°C, the column oven was set to 40°C, and the detector was set to a measurement wavelength of 254 nm. A sample solution adjusted to 1.0 mg/mL was injected at 2.0 µL, and analysis was performed in the isocratic mode. The chart after the measurement was analyzed, and the purity of delamanid was expressed as an area percentage. Table 6 shows the results.

**Table 6**

| | Purity of delamanid | |
|---|---|---|
| | Before the start of test | 60°C, after 2 weeks |
| Example A8 | 99.5% | 91.5% |
| Example A9 | 99.7% | 98.5% |
| Example A10 | 99.2% | 99.3% |

Table 6 indicates that the addition of dl-α-tocopherol improves the storage stability of delamanid at high temperatures.

### Test Example 3: Serum Concentration in Dogs

A gelatin capsule filled with the particles of Example C at a delamanid content of 50 mg was forcibly administered orally to male beagle dogs (about 30 months old, weighing 8 to 12 kg, Northern Beagles, Narc Co., Ltd.), followed immediately by forcible administration of 40 mL of water. Thirty minutes before the administration, the dogs were fed about 50 g of feed CD-5 (CLEA Japan, Inc.) and were fasted until the final blood collection. A similar study was also performed using a commercially available tablet containing 50 mg of delamanid (Deltyba (registered trademark) tablet, 50 mg) filled in a gelatin capsule.

Blood was collected before the administration and 1, 2, 4, 6, 8, 12, and 24 hours after the administration, with the amount of blood collected being about 1 mL (n=6). The obtained blood was centrifuged at 3000 rpm for 10 minutes to obtain serum. The concentration of delamanid in the serum was measured by LC-MS. Fig. 1 shows the results.

### Formulation Examples 1 to 16

Tables 7 and 8 show the formulations of Formulation Examples 1 to 16.

**Table 7**

| | Formulation Example 1 (mg) | Formulation Example 2 (mg) | Formulation Example 3 (mg) | Formulation Example 4 (mg) | Formulation Example 5 (mg) | Formulation Example 6 (mg) | Formulation Example 7 (mg) | Formulation Example 8 (mg) |
|---|---|---|---|---|---|---|---|---|
| Particles of Example C | 824 | 824 | 824 | 824 | 824 | 824 | 824 | 824 |
| Crystalline cellulose (trade name: Ceolus KG-1000,Asahi Kasei Corporation) | - | - | 50 | 100 | 50 | 50 | 50 | 100 |
| Crystalline cellulose (trade name: Ceolus UF-711 ,Asahi Kasei Corporation) | - | - | - | - | - | - | - | |
| Carmellose (trade name: NS-300, Gotoku Chemical Co., Ltd.) | 100 | 100 | 100 | 100 | 125 | 75 | 125 | 100 |
| Carmellose sodium (trade name: Kiccolate ND-2HS,Asahi Kasei Corporation) | - | - | - | - | - | - | - | - |
| Sodium starch glycolate (trade name: Primojel, DFE Pharma) | 100 | 100 | 100 | 100 | 75 | 125 | 75 | 100 |
| Crospovidone (trade name: Kollidon CL-F, BASF Japan Ltd.) | - | - | - | - | - | - | - | - |
| Carmellose calcium (trade name: ECG505, Gotoku Chemical Co., Ltd.) | - | - | - | - | - | - | - | - |
| Light anhydrous silicic acid (trade name:Adsolider 101, Freund Corporation) | 10 | 10 | 10 | 10 | 10 | 10 | 10 | 10 |
| Calcium silicate (trade name: Florite R, Tomita Pharmaceutical Co., Ltd.) | - | - | - | - | - | - | 10 | - |
| Magnesium stearate (Taihei Chemical Industrial Co., Ltd.) | 15 | 5 | 15 | 15 | 5 | 5 | 5 | 5 |

**Table 8**

| | Formulation Example 9 (mg) | Formulation Example 10 (mg) | Formulation Example 11 (mg) | Formulation Example 12 (mg) | Formulation Example 13 (mg) | Formulation Example 14 (mg) | Formulation Example 15 (mg) | Formulation Example 16 (mg) |
|---|---|---|---|---|---|---|---|---|
| Particles of Example C | 824 | 824 | 824 | 824 | 824 | 824 | 824 | 824 |
| Crystalline cellulose (trade name: Ceolus KG-1000,Asahi Kasei Corporation) | 100 | 50 | 50 | 50 | 50 | 50 | - | - |
| Crystalline cellulose (trade name: Ceolus UF-711 ,Asahi Kasei Corporation) | - | - | - | - | - | - | 75 | 100 |
| Carmellose (trade name: NS-300, Gotoku Chemical Co., Ltd.) | 100 | 200 | - | - | - | - | 100 | 100 |
| Carmellose sodium (trade name: Kiccolate ND-2HS,Asahi Kasei Corporation) | - | - | 200 | - | - | - | - | - |
| Sodium starch glycolate (trade name: Primoiel, DFE Pharma) | 50 | - | - | 200 | - | - | 50 | 50 |
| Crospovidone (trade name: Kollidon CL-F, BASFJapan Ltd.) | - | - | - | - | 200 | - | - | - |
| Carmellose calcium (trade name: ECG505, Gotoku Chemical Co., Ltd.) | - | - | - | - | - | 200 | - | - |
| Light anhydrous silicic acid (trade name:Adsolider 101, Freund Corporation) | 10 | 10 | 10 | 10 | 10 | 10 | 10 | 10 |
| Calcium silicate (trade name: Florite R, Tomita Pharmaceutical Co., Ltd.) | - | - | - | - | - | - | - | - |
| Magnesium stearate (Taihei Chemical Industrial Co., Ltd.) | 2.5 | 5 | 5 | 5 | 5 | 5 | 2.5 | 2.5 |

According to the formulations shown in Tables 7 and 8, the components other than magnesium stearate were mixed in a polyethylene bag. Thereafter, magnesium stearate was added thereto, and the mixture was further mixed. The resulting mixed powder was compressed into tablets at a tableting pressure of 20 kN using a rotary tableting machine (Clean Press, Kikusui Seisakusho Ltd.) equipped with a caplet (18.0×10.8 mm) punch to prepare caplet tablets each containing 100 mg of delamanid.

The caplet tablets of Formulation Examples 5, 7, and 16 were sprayed with a film coating liquid prepared by dissolving and suspending hydroxypropyl methylcellulose, PEG6000, titanium oxide, talc, and yellow ferric oxide in purified water according to the formulation shown in Table 9 in a film coater (Powrex Coater PRC-GTXmini, Powrex Corporation) to prepare film-coated tablets.

**Table 9**

| | (parts by mass) |
|---|---|
| Hydroxypropyl methylcellulose (trade name: TC-5E, Shin-Etsu Chemical Co., Ltd.) | 18 |
| PEG6000 (trade name: Macrogol 6000, Sanvo Chemical Industries, Ltd.) | 3 |
| Titanium oxide (trade name: Titanium oxide (IV), Merck & Co., Inc. ) | 5.7 |
| Talc (trade name: Hi-Filler No. 17, Matsumura Sangyo Co., Ltd.) | 3 |
| Yellow ferric oxide (trade name: Euroxide Yellow E7056, Sensient Pharmaceutical) | 0.3 |

## Claims

1. A solid dispersion comprising the following components (a), (b), and (c):
(a) delamanid;
(b) at least one member selected from the group consisting of polyvinyl caprolactam-polyvinyl acetate-polyethylene glycol graft copolymers and vinyl pyrrolidone-vinyl acetate copolymers; and
(c) at least one member selected from the group consisting of hydroxypropyl methylcellulose phthalates and hydroxypropyl methylcellulose acetate succinates.

2. The solid dispersion according to claim 1, wherein the mass ratio of component (a) to component (b) is within the range of 1:1 to 1:20.

3. The solid dispersion according to claim 1, wherein the mass ratio of component (a) to component (b) is within the range of 1:1 to 1:8.

4. The solid dispersion according to claim 1, wherein the mass ratio of component (a) to component (b) is within the range of 1:1 to 1:5.

5. The solid dispersion according to claim 1, wherein the mass ratio of component (a) to component (c) is within the range of 1:1 to 1:10.

6. The solid dispersion according to claim 1, wherein the mass ratio of component (a) to component (c) is within the range of 1:1 to 1:8.

7. The solid dispersion according to claim 1, wherein the mass ratio of component (a) to component (c) is within the range of 1:1 to 1:5.

8. The solid dispersion according to claim 1, wherein the mass ratio of component (a) to component (b) is within the range of 1:1 to 1:5, and the mass ratio of component (a) to component (c) is within the range of 1:1 to 1:5.

9. The solid dispersion according to claim 1, wherein component (b) comprises a polyvinyl caprolactam-polyvinyl acetate-polyethylene glycol graft copolymer.

10. The solid dispersion according to claim 1, wherein component (b) comprises a vinyl pyrrolidone-vinyl acetate copolymer.

11. The solid dispersion according to claim 1, wherein component (c) comprises a hydroxypropyl methylcellulose phthalate.

12. The solid dispersion according to claim 1, wherein component (b) comprises a polyvinyl caprolactam-polyvinyl acetate-polyethylene glycol graft copolymer, and component (c) comprises a hydroxypropyl methylcellulose phthalate.

13. The solid dispersion according to claim 1, wherein component (b) comprises a vinyl pyrrolidone-vinyl acetate copolymer, and component (c) comprises a hydroxypropyl methylcellulose phthalate.

14. The solid dispersion according to claim 1, further comprising vitamin E.

15. The solid dispersion according to claim 14, wherein the vitamin E is dl-α-tocopherol.

16. The solid dispersion according to any one of claims 1 to 15, which is a hot-melt extrudate or a ground material thereof.

17. A pharmaceutical composition comprising the solid dispersion of any one of claims 1 to 16.

18. An oral solid formulation comprising the solid dispersion of any one of claims 1 to 16.

19. The oral solid formulation according to claim 18, which is a tablet or a capsule.

20. A method for producing the solid dispersion of any one of claims 1 to 16, comprising subjecting a composition comprising components (a), (b), and (c) to hot-melt extrusion.
